# EUROPEAN PATENT SPECIFICATION

(11) **EP 1 858 563 B1**
(45) Date of publication and mention of the grant of the patent: **27.07.2016**
(21) Application number: 05707699.4
(22) Date of filing: 03.03.2005
(51) Int. Cl.: A61L 29/14, A61L 29/04, A61L 33/00

(54) **MEDICAL DEVICE HAVING ANTITHROMBOTIC AND LUBRICIOUS PROPERTIES**
MEDIZINPRODUKT MIT ANTITHROMBOTISCHEN UND SCHMIERENDEN EIGENSCHAFTEN
DISPOSITIF MÉDICAL DE TRAITEMENT ET SON PROCÉDÉ DE FABRICATION

(43) Date of publication of application: 28.11.2007
(73) Proprietor: Covidien AG, 8212 Neuhausen am Rheinfall (CH)
(72) Inventor: AMANO, Kenichi, Fukuroi-shi, Shizuoka 437-0004 (JP); SANO, Tomokazu, Fukuroi-shi, Shizuoka 437-0004 (JP); NISHITANI, Kyoko, Fukuroi-shi, Shizuoka 437-0004 (JP)
(74) Representative: Tomlinson, Edward James
(86) International application number: PCT/EP2005/002258
(87) International publication number: WO 2006/094521

(56) References cited:
- EP-A- 0 693 293
- US-A- 5 069 899
- US-A- 5 135 516
- US-A1- 2002 016 574

## Description

### [Technical Field]

The present invention relates to a medical treatment device wherein a coating having an antithrombotic property and Lubricity is formed on the surface of a catheter, for example, and also a method for manufacturing the same.

### [Background Art]

Among medical treatment devices are those which are punctured into and left to remain in blood vessels such as catheters or guide needles used by inserting into a catheter Since such medical treatment devices are foreign substances to the blood, when the medical treatment device makes contact with blood in blood vessel, a blood coagulation reaction may take place and a thrombus block may be formed, As a result of such formation of thrombus blocks, there are some cases in which not only is the original objective, treatment or diagnosis of diseases, not achieved but new complications are generated by the thrombus which may be severe. Therefore, antithrombotic property is required for the medical device contacting blood in the blood vessel.

With regard to a method for the manufacture of such medical devices having an antithrombotic property, there is a method whereby a coating of a copolymer of methyl vinyl ether with maleic acid anhydride which is to be a binder for polyurethane and urokinase is formed on the surface of a substrate comprising polyurethane and then urokinase is contained in the coating by means of impregnation or the like (refer, for example, to Patent Document 1). In addition, lubricity is required of such a medical treatment device for preventing damage to mucous membranes or for attenuating pain in patients when the device is inserted into the body. With regard to such medical treatment devices having lubricity, there is a product in which a fibrinolysis activating substance is bonded to a cross-linked coating comprising polyol and polymer having an acid anhydride group whereby lubricity is expressed upon being moisturized (refer, for example, to Patent Document 2).
Patent Document 1: Japanese Patent Laid-Open No. 63/98,384
Patent Document 2: Japanese Patent Laid-Open No. 10/248, 918

US 5,135,516 describes a method for rendering a medical device lubricious and antithrombogenic by coating the device with a thin coating of a lubricious polymer including acid groups (e.g. carboxyl groups) and applying ammonium cation (e.g. quaternary ammonium salt) and heparin and thereafter applying a buffer solution.

### [Disclosure of the Invention]

Among the medical treatment devices as mentioned above, there is a catheter which is used in a state of being retained in a blood vessel of a patient for as long as one month. However, a coating for expressing an antithrombotic property in the above-mentioned conventional medical treatment device has insufficient durability and could not be used for a long period. In addition, lubricity could not be maintained for a long period.

The present invention has been achieved in view of such circumstances and its object is to provide a medical treatment device wherein the antithrombotic property and lubricity of a coating formed on the surface can be maintained in a stable state for a long period and also to provide a method for manufacturing the same

In order to achieve the above-mentioned object, characteristic features of the medical treatment device concerning the present invention are that a fibrinolysis activating enzyme fixer for fixation of fibrinolysis activating enzyme, a suppressor fixer for fixation of suppressor which suppresses blood coagulation factor activity and a fixing reinforcer for reinforcing fixing action of the fibrinolysis activating enzyme fixer and the suppressor fixer, are dissolved in an organic solvent to manufacture a mixture solution for coating, the mixture solution for coating is applied on the surface of a substrate comprising synthetic resin to form a coating and, at the same time, the fibrinolysis activating enzyme, the suppressor or both the fibrinolysis activating enzyme and the suppressor are combined with the coating.

In the thus constituted medical treatment device, the coating formed on the surface of the substrate contains a fibrinolysis activating enzyme bonded to the substrate by a fibrinolysis activating enzyme fixer, a suppressor which is bonded to the substrate by a suppressor fixer or both the fibrinolysis activating enzyme and the suppressor. Both the fibrinolysis activating enzyme and the suppressor have an antithrombotic property. In addition, the coating contains a fixation promoter which enhances the durability of the antithrombotic property by strengthening the close contact of both the fibrinolysis activating enzyme fixer and the suppressor fixer to the coating. Accordingly, in the medical treatment device of the present invention, a synergistic effect is achieved due to the use of both a fibrinolysis activating enzyme and a suppressor whereby far better antithrombotic property and durability are attained compared to the antithrombotic property and durability of the medical device having a coating containing either the fibrinolysis activating enzyme or the suppressor but not both. Incidentally, even when either the fibrinolysis activating enzyme or the suppressor is used but not both together, an excellent effect can be achieved as well.

Another compositional characteristic of the medical treatment device according to the present invention is that an alkaline treatment comprising impregnation in an alkaline solution is applied to a predetermined part of a substrate on which a coating has been formed by application of the mixture solution for coating. This alkaline treatment of a coating formed on a substrate, on the surface of the medical treatment device, is carried out so that lubricity as generated when the device is moisturized. The alkaline treatment at that time is conducted by impregnation of the formed coating in an alkaline solution such as sodium hydroxide or potassium hydroxide for a predetermined time. The alkaline treatment need be applied only to the part of the medical treatment device which needs to have lubricity For example, when the medical treatment device is a catheter, the treatment may be conducted to all of the parts which are inserted into body or may be conducted to the leading end part only.

With regard to a fixer for a fibrinolysis activating enzyme, a copolymer of methyl vinyl ether with maleic acid anhydride may be used while, with regard to a fixing reinforcer for strengthening refixing action, a polyether block amide may be used. The suppressor fixer may be a quaternary ammonium salt, and the quaternary ammonium salt may be tridodecylmethyl ammonium chloride. Since, among the quaternary ammonium salts tridodecylmethylammonium chloride is characterized by excellent close adhesion to synthetic resins, it is able to achieve a particularly pronounced effect

The fibrinolysis activating enzyme may be urokinase, streptokinase, tissue plasminogen activator, plasmin or prinolase. The suppressor may be heparin, hirudin, thrombomodulin or an antiplatelet substance.

Still another constitutional characteristic of the medical treatment device according to the present invention is that the synthetic resin constituting the substrate is polyurethane, polyvinyl chloride, nylon or nylon elastomer. As a result, affinity between the substrate and the fixing reinforcer for strengthening the fixing agent, such as polyether block amide, increases, whereby the antithrombotic property and durability of the formed coating are improved.

A compositional characteristic of a method for the manufacture of a medical treatment device according to the present invention is that it comprises a step for the manufacture of a mixture solution for coating in which a fibrinolysis activating enzyme fixer for fixation of fibrinolysis activating enzyme, a suppressor fixer for fixation of suppressor which suppresses blood coagulation factor activity and a fixing reinforcer for strengthening fixing action of the fibrinolysis activating enzyme fixer and the suppressor fixer are dissolved in an organic solvent, a step for the formation of a coating in which the mixture solution for coating is applied on the surface of a substrate comprising a synthetic resin to form a coating and a step for impregnation in which the fibrinolysis activating enzyme, the suppressor or both the fibrinolysis activating enzyme and the suppressor are impregnated in the coating.

After the coating formation step, it is also possible to add an alkaline treatment step wherein a predetermined part of the substrate whereupon the coating is formed is immersed in an alkaline solution. As a result, not only an antithrombotic property but also Lubricity can be achieved in the medical treatment device. In such a case, a copolymer of methyl vinyl ether with maleic acid anhydride may be used as a fixer for fibrinolysis activating enzyme, a quaternary ammonium salt may be used as a fixer for suppressor, a polyether block amide may be used as a fixing reinforcer for fixing action, urokinase may be used as a fibrinolysis activating enzyme and heparin may be used as a suppressor. As a result, a coating having an antithrombotic property on the substrate surface can be formed in a stable manner by a simple treatment step which thus can be applied to actual mass-production

Another constitutional characteristic of the medical devices according to the present invention is that, after the coating step formation, there is provided a drying step where the coating is subjected to a drying treatment at a temperature range from room temperature to 80°C. As a result, a coating having sufficient and stable antithrombotic property can be formed by a simple treatment step which in addition can be applied to various medical treatment devices which are deformed by a high temperature treatment, such as catheters.

Another constitutional characteristic of the method for the manufacture of medical treatment device according to the present invention is that, after the step for impregnation, there is provided a sterilizing step where the coating is sterilized by gamma radiation. The sterilization by gamma radiation acts upon molecules of the substance directly, without utilisation of heat and chemical reaction and, therefore, safe and sure sterilization can be efficiently carried out. As a result, urokinase can be fixed to the medical treatment device while sufficiently retaining the effectiveness of the urokinase, so that it is possible to manufacture a safer medical treatment device as well

### [Best Mode for Carrying Out the Invention]

In the medical treatment device according to the present invention, a substrate comprising a synthetic resin is immersed in a mixture solution for coating which is prepared by dissolving a copolymer of methyl vinyl ether with maleic acid anhydride, a quaternary ammonium salt and a polyether block amide in an organic solvent, whereby a coating is formed on the surface of the substrate. When the substrate upon which the coating is formed is immersed in an aqueous solution containing urokinase and heparin, urokinase and heparin is incorporated into the coating.

In this coating, urokinase and heparin are present for a long period and antithrombotic property and durability thereof are effectively achieved. Also, after a coating is formed on the substrate surface, a predetermined part of the coating can be immersed in an alkaline solution for a predetermined time to conduct an alkaline treatment. As a result, lubricity can be given to the coating. Antithrombotic property, lubricity and durability thereof in the medical treatment device prepared as such will be illustrated in the following Examples 1 and 2.

### [Example 1]

Tridodecylmethylammonium chloride (trade name: Tridodecylmethylammonium Chloride, manufactured by Polysciences; hereinafter referred to as "C") was added to a mixture solution wherein a 2% solution of a copolymer of methyl vinyl ether with maleic acid anhydride (trade name: Gantrez AN-159, manufactured by ISP (international Specialty Products) ; hereinafter referred to as "A") in acetone was mixed with a 2% solution of a polyether block amide (trade name: Pebax 2533 SA, manufactured by Atochem; hereinafter referred to as "B") in THF in a ratio of 1.5:1, whereby A:B:C was made 3:2:1, to prepare a mixture solution for coating.

A substrate was immersed in this mixture solution for coating, removed, dried under reduced pressure for 3 hours at a drying temperature of 60°C, immersed at 5°C for 24 hours into a 0.7% solution of heparin sodium (manufactured by Diosynth) mixed in acidic physiological saline (pH = 4..6) containing 300 IU/ml of urokinase (manufactured by JCR), removed and dried *in* vacuo to form a coating on the surface of the substrate. The substrate surface including the coating was irradi ated with 40 kGY of gamma radiation for sterilisation.

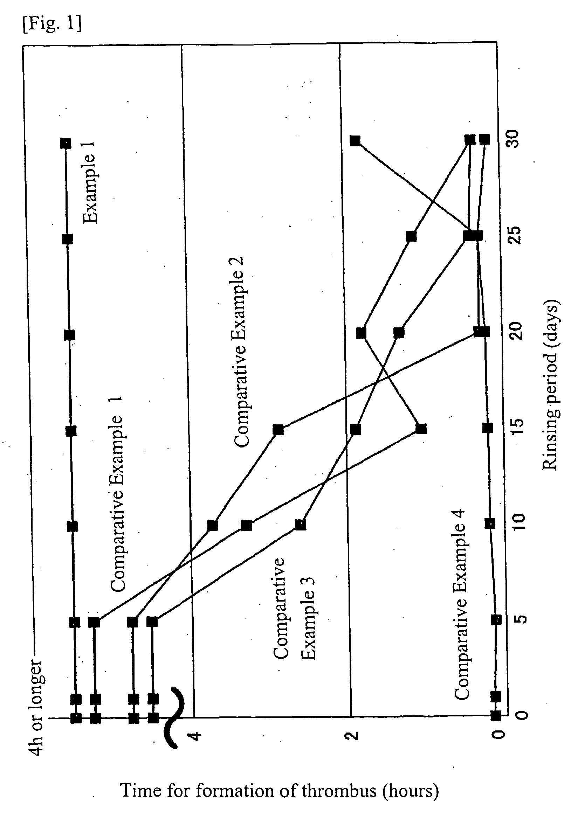

The substrate used was a tube made of polyurethane (trade name: Tecoflex; manufactured by Thermedics), of which the diameter was 14G and the full length was 20 cm. The coating formed in the inner area of the tube was subjected to the following antithrombotic test, The same antithrombotic test was carried out for the coating formed on the inner coating of a medical treatment device according to the Comparative Examples and also for the tube itself. The result of those tests is shown in Fig. 1.

In the antithrombotic test, an aqueous solution of Na⁺ and Ca²⁺ in the same concentrations as in plasma (wherein 8,307 mg of NaCl and 278 mg of CaCl₂ were dissolved in 1 liter of distilled water) and kept at 37°C, the same as body temperature was prepared. The aqueous solution was passed into an inner area of the tube at a flow rate of 20 ml/hour for 0, 1, 5, 10, 15, 20, 25 or 30 days to rinse the coating formed in the inner area. After that, 0.1 ml of human whole blood was placed into the inner area of the tube at room temperature and both ends of the tube was connected and sealed to form a loop.

The tube loop was rotated at the rate of 5 r.p.m. and the time it took for the blood to lose fluidity was measured as equivalent to the time for thrombus generation. In this operation, while the blood remained at the lower side of the rotating tube loop, it was judged that the blood had fluidity, and when blood began to rotate together with the tube loop, it was judged that fluidity was lost For determining each rinsing time, n=5 data on the whole blood of five persons was averaged.

In Fig. 1. the abscissa shows the period of rinsing é(day(s)) and the ordinate shows time (hour(s)) for generation of thrombus. In Comparative Examples 1 and 2, a commercially available product wherein urokinase was fixed on the surface of the tube was used and, in Comparative Example 3, the same wherein heparin sodium was fixed on the surface of the tube was used. In Comparative Example 4, a product wherein no coating had been formed on the surface of the tube was used.

As will be apparent from the test result shown in Fig. 1, in the case of Example 1, blood retained fluidity and no thrombus was generated even after 4 hours from the initiation of the test in all cases in which the rinsing period was from 0 to 30 day(s). On the contrary, in Comparative Examples 1 to 3, blood retained fluidity and no thrombus was generated even after 4 hours from the initiation of the test when the rinsing period was 0 to 5 day(s) while, when the rinsing period was 10 to 30 days, blood lost fluidity and thrombus was generated within 4 hours from initiation of the test. In addition, in these cases, there was noted the tendency that the longer the rinsing period, the shorter the thrombus generation time. In Comparative Example 4, thrombus was generated within 20 minutes from the initiation of the test in all cases in which the rinsing period was from 0 to 30 day(s).

From these results, it is concluded that, in Example 1, effective amounts of urokinase and heparin remained in the coating whereby the antithrombotic property was maintained in any of the cases in which the rinsing period was 0 to 30 day(s). In Comparative Examples 1 to 3, it was presumed that effective amounts of urokinase and heparin remained in the coating, that is, antithrombotic property was maintained, when the rinsing period was 1 to 5 day(s) while, when the rinsing period was 10 to 30 days, it was concluded that an effective amount of urokinase or heparin did not remain in the coating. On the basis of these results, it is presumed that the longer the rinsing period, the less the residual amounts of urokinase and heparin.

From the above results, it may be concluded that the medical treatment device according to Example 1 is superior to any of the medical treatment devices of Comparative Examples 1 to 4 in the degree and durability of antithrombotic property That is because both urokinase and heparin are fixed to the substrate by a methyl vinyl ether/maleic acid anhydride copolymer and tridodecylmethylammonium chloride, respectively, and further because the fixation is reinforced by a polyether block amide. As a result, it is now possible to prepare a medical treatment device wherein a durable antithrombotic property of the surface is achieved.

### [Example 2]

The substrate in the step for the manufacture of the test sample for Example 1 was immersed in a mixture solution for coating, dried under reduced pressure at 60°C for 3 hours and subjected to an alkaline treatment wherein the lubricated part of the substrate was immersed for 3 minutes in a 0 .1N aqueous solution of sodium hydroxide. After the substrate was removed from the sodium hydroxide solution, it was dried under reduced pressure at 60°C for 3 hours. All other steps were the same as those in the manufacture of the test sample for Example 1 to prepare the test sample which will be called Example 2. The test for surface lubricity was conducted for the test samples of Example 2 and the above-mentioned Comparative Examples 1 to 4 The result is shown in the following Table 1.

**[Table 1]**

| | Initial Lubricity | Lubricity after 50 Rubbings | Lubricity after Immersion in Warm Water |
|---|---|---|---|
| Example 2 | OO | OO | OO |
| Comparative Examples 1 to 4 | × | × | × |

The surface lubricity test was conducted on the basis of feeling or touching, the surface of each test sample being touched by the finger. The case in which lubricity was good was marked "OO", the case in which some lubricity was noted was marked "o" (in the present test, such a result was not obtained) and the case in which lubricity was poor was marked "×". The above surface lubricity test was carried out for the initial state, after 50 rubbings and after immersion in warm water. The initial lubricity was tested by feeling with the finger a test sample that was unchanged since manufacture, and the lubricity after 50 rubbings was tested by feeling with the finger a test sample that had been wetted with physiological saline and rubbed by the finger 50 times. The lubricity after dipping in warm water was tested by feeling with the finger after the test sample had been immersed in a physiological saline of 50°C for 24 hours.

As shown in Table 1, the result was that, in Example 2, lubricity was good in all cases while, in Comparative Examples 1 to 4, lubricity was poor in all cases. From the above, it is concluded that, when a coating on the substrate is subjected to an alkaline treatment, a good lubricity can be achieved and further that the lubricity can be maintained for long time.. With regard to the antithrombotic property, the test sample of Example 2 shows the same effect, to the same extent, as the test sample of Example 1.

As mentioned above, in the medical treatment device according to the present invention, there is a coating prepared by application of a mixture solution for coating, comprising a copolymer of methyl vinyl ether with maleic acid anhydride, a quaternary ammonium salt and a polyether block amide dissolved in an organic solvent, on the surface of a substrate comprising a synthetic resin and also by impregnating urokinase and heparin therein, whereby an excellent antithrombotic property is achieved In addition, urokinase and heparin contained in the coating are present in the coating for a long period and, therefore, the antithrombotic property is maintained for a long period. When an alkaline treatment is further applied to a predetermined part of the surface of the substrate, a good lubricity can be achieved for a long period. As a result, it is possible to produce a medical treatment device of great practical value.

Incidentally, the present invention is not limited to the above-mentioned Examples and can be modified. For example, with regard to a fibrinolysis activating enzyme, it is also possible to use streptokinase, tissue plasminogen activator, plasmin, prinolase, etc. instead of urokinase With regard to a suppressor for suppressing blood coagulation, it is possible to use hirudin, thrombomodulin, antiplatelet substance, etc, instead of heparin. With regard to a synthetic resin comprising the substrate, it is also possible to use polyvinyl chloride, nylon, nylon elastomer, etc, instead of polyurethane. With regard to an alkaline aqueous solution, it is also possible to use potassium hydroxide, etc. instead of sodium hydroxide.

### [Brief Description of the Drawing]

Fig 1 is a graph which shows the relation between thrombus formation time and rinsing period.

## Claims

1. A medical treatment device comprising:
a substrate comprising a synthetic resin; and
a coating on a surface of the substrate, the coating comprising either or both of:
(i) a fibrinolysis activating enzyme selected from the group consisting of urokinase, streptokinase, tissue plasminogen activator, and prinolase, and
(ii) a suppressor selected from the group consisting of heparin, hirudin, thrombomodulin, and antiplatelet agents for suppressing blood coagulation factor activity
in combination with a a copolymer of methyl vinyl ether with maleic acid anhydride as a fibrinolysis activating enzyme fixer for fixation of the fibrinolysis activating enzyme, a quaternaary ammonium salt as a suppressor fixer for fixation of the suppressor which suppresses blood coagulation factor activity, and
a polyether block amide as a fixing reinforcer for reinforcing the fixing action of the fibrinolysis activating enzyme fixer and the suppressor fixer.

2. The medical treatment device according to claim 1, wherein a portion of the coating is impregnated with an alkaline solution.

3. The medical treatment device according to claim 1, wherein the quaternary ammonium salt is tridodecylmethylammonium chloride.

4. The medical treatment device according to claim 1, wherein the synthetic resin is selected from the group consisting of polyurethane, polyvinyl chloride, nylon, and nylon elastomers.

5. A method for the manufacture of a medical treatment device comprising:
providing a medical treatment device comprising a substrate comprising a synthetic resin having a surface;
forming a solution for a coating by combining
in an organic solvent a copolymer of methyl vinyl ether with maleic acid anhydride as a fibrinolysis activating enzyme fixer for fixation of a fibrinolysis activating enzyme, a quaternary ammonium salt as a suppressor fixer for fixation of a suppressor which suppresses blood coagulation factor activity and a polyether block amide as a fixing reinforcer for reinforcing fixing action of the fibrinolysis activating enzyme fixer and the suppressor fixer;
applying the coating solution to the surface of the substrate; and obtaining a medical device possessing a coating on its surface; and
impregnating the coating with one or both of the fibrinolysis activating enzyme and the suppressor, the fibrinolysis activating enzyme selected from the group consisting of urokinase, streptokinase, tissue plasminogen activator, plasmin, and prinolase and the suppressor selected from the group consisting of heparin, hirudin, thrombomodulin. and antiplatelet agents.

6. The method according to claim 5, further comprising subjecting a portion of the coating to an alkaline treatment by immersion in an alkaline solution.

7. The method according to claim 5, further comprising drying the coating at a temperature from room temperature to 80°C.

8. The method according to claim 5, further comprising sterilizing the coating by exposing the coating to gamma radiation.

9. The method according to claim 5, wherein the quaternary ammonium salt is tridodecylmethylammonium chloride.

10. The method according to claim 5, wherein the substrate comprises a synthetic resin selected from the group consisting of polyurethane, polyvinyl chloride, nylon, and nylon elastomers.

## Patentansprüche

1. Medizinische Behandlungsvorrichtung, umfassend:
ein Substrat, das ein synthetisches Harz umfasst; und
eine Beschichtung auf einer Oberfläche des Substrats, wobei die Beschichtung eines oder beides umfasst von:
(i) einem Fibrinolyse-aktivierenden Enzym, das ausgewählt ist aus der Gruppe bestehend aus Urokinase, Streptokinase, gewebespezifischem Plasminogenaktivator und Prinolase, und
(ii) einem Hemmmittel, das ausgewählt ist aus der Gruppe bestehend aus Heparin, Hirudin, Thrombomodulin und Thrombozytenaggregationshemmern, zur Hemmung der Blutgerinnungsfaktoraktivität
in Kombination mit einem Copolymer von Methylvinylether mit Maleinsäureanhydrid als Fibrinolyse-aktivierender Enzymfestiger zur Festigung des Fibrinolyse-aktivierenden Enzyms, einem quaternären Ammoniumsalz als Hemmmittelfestiger zur Festigung des Hemmmittels, das die Blutgerinnungsfaktoraktivität hemmt, und
einem Polyetherblockamid als Festigungsverstärker zur Verstärkung der Festigungswirkung des Festigers des Fibrinolyse-aktivierenden Enzyms und des Hemmmittelfestigers.

2. Medizinische Behandlungsvorrichtung nach Anspruch 1, wobei ein Teil der Beschichtung mit einer Alkalinlösung imprägniert ist.

3. Medizinische Behandlungsvorrichtung nach Anspruch 1, wobei es sich bei dem quaternären Ammoniumsalz um Tridodecylmethylammoniumchlorid handelt.

4. Medizinische Behandlungsvorrichtung nach Anspruch 1, wobei das synthetische Harz ausgewählt ist aus der Gruppe bestehend aus Polyurethan, Polyvinylchlorid, Nylon und Nylonelastometern.

5. Verfahren für die Fertigung einer medizinischen Behandlungsvorrichtung, umfassend:
Bereitstellen einer medizinischen Behandlungsvorrichtung, welche ein Substrat umfasst, das ein synthetisches Harz mit einer Oberfläche aufweist;
Bilden einer Lösung für eine Beschichtung durch Kombinieren
eines Copolymers von Methylvinylether mit Maleinsäureanhydrid als Festiger des Fibrinolyse-aktivierenden Enzyms, einem quaternären Ammoniumsalz als Hemmmittelfestiger zur Festigung eines Hemmmittels, das die Blutgerinnungsfaktoraktivität hemmt, und einem Polyetherblockamid als Festigungsverstärker zum Verstärken der Festigungswirkung des Festigers des Fibrinolyse-aktivierenden Enzyms und des Hemmmittelfestigers in einer organischen Lösung;
Auftragen der Beschichtungslösung auf die Oberfläche des Substrats; und Erhalt einer medizinischen Vorrichtung, die eine Schicht auf ihrer Oberfläche besitzt; und
Imprägnieren der Beschichtung mit einem oder beiden von dem Fibrinolyse-aktivierenden Enzym und dem Hemmmittel, wobei das Fibrinolyse-aktivierende Enzym ausgewählt ist aus der Gruppe bestehend aus Urokinase, Streptokinase, gewebespezifischem Plasminogenaktivator, Plasmin und Prinolase und das Hemmmittel ausgewählt ist aus der Gruppe bestehend aus Heparin, Hirudin, Thrombomodulin und Thrombozytenaggregationshemmern.

6. Verfahren nach Anspruch 5, weiter umfassend das Unterziehen eines Teils der Beschichtung einer alkalischen Behandlung durch Eintauchen in eine alkalische Lösung.

7. Verfahren nach Anspruch 5, weiter umfassend das Trocknen der Beschichtung bei einer Temperatur zwischen der Raumtemperator und 80°C.

8. Verfahren nach Anspruch 5, weiter umfassend das Sterilisieren der Beschichtung, indem die Beschichtung einer Gammastrahlung ausgesetzt wird.

9. Verfahren nach Anspruch 5, wobei es sich bei dem quaternären Ammoniumsalz um Tridodecylmethylammoniumchlorid handelt.

10. Verfahren nach Anspruch 5, wobei das Substrat ein synthetisches Harz umfasst, das ausgewählt ist aus der Gruppe bestehend aus Polyurethan, Polyvinylchlorid, Nylon und Nylonelastometern.

## Revendications

1. Dispositif de traitement médical comprenant :
un substrat comprenant une résine synthétique ; et
un revêtement sur une surface du substrat, le revêtement comprenant un ou les deux de :
(i) une enzyme d'activation de la fibrinolyse choisie dans le groupe constitué par l'urokinase, la streptokinase, l'activateur tissulaire du plasminogène et la prinolase, et
(ii) un suppresseur choisi dans le groupe constitué par l'héparine, l'hirudine, la thrombomoduline et les agents antiplaquettaires pour la suppression de l'activité du facteur de coagulation du sang en combinaison avec un un copolymère d'éther méthyl-vinylique avec l'anhydride de l'acide maléique comme fixateur de l'enzyme d'activation de la fibrinolyse pour la fixation de l'enzyme d'activation de la fibrinolyse, un sel d'ammonium quaternaire comme fixateur suppresseur pour la fixation du suppresseur qui supprime l'activité du facteur de coagulation du sang, et un polyéther bloc amide comme renforçateur de fixation destiné à renforcer l'action de fixation du fixateur de l'enzyme d'activation de la fibrinolyse et du fixateur suppresseur.

2. Dispositif de traitement médical selon la revendication 1, dans lequel une partie du revêtement est imprégnée d'une solution alcaline.

3. Dispositif de traitement médical selon la revendication 1, dans lequel le sel d'ammonium quaternaire est du chlorure de tridodécylméthylammonium.

4. Dispositif de traitement médical selon la revendication 1, dans lequel la résine synthétique est choisie dans le groupe constitué du polyuréthane, du chlorure de polyvinyle, du nylon et d'élastomères de nylon.

5. Procédé pour la fabrication d'un dispositif de traitement médical, comprenant :
la fourniture d'un dispositif de traitement médical comprenant un substrat comprenant une résine synthétique ayant une surface ;
la formation d'une solution pour un revêtement en combinant dans un solvant organique un copolymère d'éther méthyl-vinylique avec de l'anhydride de l'acide maléique comme fixateur de l'enzyme d'activation de la fibrinolyse pour la fixation d'une enzyme d'activation de la fibrinolyse, un sel d'ammonium quaternaire comme fixateur suppresseur pour la fixation d'un suppresseur qui supprime l'activité du facteur de coagulation du sang et un polyéther bloc amide comme renforçateur de fixation destiné à renforcer l'action de fixation du fixateur de l'enzyme d'activation de la fibrinolyse et du fixateur suppresseur ;
l'application de la solution de revêtement sur la surface du substrat ; etl'obtention d'un dispositif médical présentant un revêtement sur sa surface ; et
l'imprégnation du revêtement avec un ou les deux de l'enzyme d'activation de la fibrinolyse et du suppresseur, l'enzyme d'activation de la fibrinolyse étant choisie dans le groupe constitué par l'urokinase, la streptokinase, l'activateur tissulaire du plasminogène, la plasmine et le prinolase, et le suppresseur étant choisi dans le groupe constitué par l'héparine, l'hirudine, la thrombomoduline et les agents antiplaquettaires.

6. Procédé selon la revendication 5, comprenant en outre la soumission d'une partie du revêtement à un traitement alcalin par immersion dans une solution alcaline.

7. Procédé selon la revendication 5, comprenant en outre le séchage du revêtement à une température allant de la température ambiante à 80 °C.

8. Procédé selon la revendication 5, comprenant en outre la stérilisation du revêtement en exposant le revêtement à un rayonnement gamma.

9. Procédé selon la revendication 5, dans lequel le sel d'ammonium quaternaire est du chlorure de tridodécylméthylammonium.

10. Procédé selon la revendication 5, dans lequel le substrat comprend une résine synthétique choisie dans le groupe constitué du polyuréthane, du chlorure de polyvinyle, du nylon et d'élastomères de nylon.
